# EUROPEAN PATENT SPECIFICATION

(11) **EP 3 369 826 B1**
(45) Date of publication and mention of the grant of the patent: **07.12.2022**
(21) Application number: 16860046.8
(22) Date of filing: 15.04.2016
(51) Int. Cl.: C12Q 1/6886, G01N 33/574

(54) **BIOMARKER MICRORNA FOR PREDICTION OF PROGNOSIS OF HEAD AND NECK CANCER**
BIOMARKER-MIKRORNA ZUR VORHERSAGE DER PROGNOSE VON KOPF- UND HALSKREBS
MICROARN BIOMARQUEUR POUR LA PRÉDICTION DU PRONOSTIC D'UN CANCER DE LA TÊTE ET DU COU

(30) Priority: 30.10.2015 KR 20150152072
(43) Date of publication of application: 05.09.2018
(73) Proprietor: The Catholic University of Korea Industry-Academic Cooperation Foundation, Seoul 06591 (KR)
(72) Inventor: KO, Yoon-Ho, Seoul 06602 (KR)
(74) Representative: Schnappauf, Georg
(86) International application number: PCT/KR2016/003941
(87) International publication number: WO 2017/073862

(56) References cited:
- WO-A1-2014/201542
- US-A1- 2013 005 599
- US-A1- 2015 018 227
- US-A1- 2015 018 227
- US-A1- 2015 024 961
- FENGWEI AN ET AL: "Subpath analysis of each subtype of head and neck cancer based on the regulatory relationship between miRNAs and biological pathways", ONCOLOGY REPORTS, vol. 34, no. 4, 24 October 2015 (2015-10-24), pages 1745-1754, XP055566098, ISSN: 1021-335X, DOI: 10.3892/or.2015.4150
- ZAHRA JAMALI ET AL: "MicroRNAs as prognostic molecular signatures in human head and neck squamous cell carcinoma: A systematic review and meta-analysis", ORAL ONCOLOGY, vol. 51, no. 4, 9 February 2015 (2015-02-09), pages 321-331, XP055316311, GB ISSN: 1368-8375, DOI: 10.1016/j.oraloncology.2015.01.008
- TAN GONGJUN ET AL: "The role of microRNAs in nasopharyngeal carcinoma", TUMOR BIOLOGY, KARGER, BASEL, CH, vol. 36, no. 1, 27 November 2014 (2014-11-27), pages 69-79, XP036217771, ISSN: 1010-4283, DOI: 10.1007/S13277-014-2847-3 [retrieved on 2014-11-27]
- MASASHI SHIIBA ET AL: "MicroRNAs in Head and Neck Squamous Cell Carcinoma (HNSCC) and Oral Squamous Cell Carcinoma (OSCC)", CANCERS, vol. 2, no. 2, 21 April 2010 (2010-04-21), pages 653-669, XP055333502, DOI: 10.3390/cancers2020653
- DE CECCO, LORIS et al.: "Head and Neck Cancer Subtypes with Biological and Clinical Relevance: Meta-analysis of Gene -expression Data", Oncotarget, vol. 6, no. 11, 20 March 2015 (2015-03-20) , pages 9627-9642, XP055379494,
- KO, YOON HO et al.: "Integrative Analysis of miRNAs Classify Two Distinct Stages of Epithelial Cell Differentiation in Head and Neck Squamous Cell Carcinoma (HNSCC", American Association for Cancer Research Annual Meeting, April 2015 (2015-04),
- KO, YOON HO et al.: "Abstract 4007 :Integrative Analysis of miRNAs Classify Two Distinct Stages of Epithelial Cell Differentiation in Head and Neck Squamous Cell Carcinoma (HNSCC", The 41nd Annual Meeting of Korean Cancer Association with International Cancer Conference, vol. 75, no. S15, April 2015 (2015-04), XP009510775, ISSN: 0008-5472, DOI: 10.1158/1538-7445.AM2015-4007
- 2 Genechip ET AL: "GeneChip miRNA Array Package Insert Instructions for Use Instructions on using the Affymetrix FlashTag (TM) Biotin HSR RNA Labeling Kit can be downloaded from Instrumentation and Software Required 1. GeneChip Scanner 3000 7G 3. GeneChip Hybridization Oven 645 Accessory Files Fluidics Scripts", , 1 January 2013 (2013-01-01), XP055748875, Retrieved from the Internet: URL:http://www.affymetrix.com/support/tech nical/byproduct.affx?product=miRNAGalaxy [retrieved on 2020-11-10]

## Description

### Technical Field

The present invention relates to a microRNA (hereinafter referred to as "miRNA") which may be used as a biomarker for predicting the prognosis of head and neck cancer.

### Background Art

Cancer is a group of diseases characterized by abnormal control of cell growth. There are over 100 different types of cancers, and each is classified by the type of cell that is initially affected. Examples of cancer include bladder cancer, breast cancer, colon cancer, rectal cancer, endometrial cancer, renal cancer (renal cell carcinoma), leukemia, small cell lung cancer, non-small cell lung cancer, pancreatic cancer, prostate cancer, thyroid cancer, skin cancer, non-Hodgkin's lymphoma, melanoma, and head and neck cancer.

In fact, head and neck cancer (HNC) is the sixth most common cancer worldwide, with an annual incidence of more than 640,000 cases worldwide. Head and neck squamous cell carcinoma (HNSCC) accounts for over 90% of all head and neck cancers, and thus is the most common form of head and neck cancer, and it occurs on the mucosal surface throughout the anatomical region. This category includes tumors of the nasal cavity, the paranasal sinuses, the oral cavity, nasopharynx, oropharynx, hypopharynx and larynx. 35% to 45% of head and neck cancer patients eventually die of head and neck squamous cell carcinoma disease. In the United States alone, head and neck squamous cell carcinoma accounts for about 4% of all malignant tumors. This corresponds to about 17 of 100,000 patients with newly diagnosed head and neck squamous cell carcinoma each year (Jemal A, Siegel R, Ward E, et al. Cancer statistics, 2008, CA Cancer J. Clin. 2008 Mar-Apr; 58(2):7196).

Head and neck cancer is a disease that causes significant morbidity, especially with respect to speech and swallowing functions. Surgery, radiotherapy, chemotherapy or combinations thereof are generally available as treatment options. However, despite rigorous combinations of surgery, chemotherapy and radiation, cure rates reach only 30% for late stage disease. Accordingly, the use of biomarkers for predicting the prognosis of head and neck cancer has been demanded.

### Disclosure

### Technical Problem

The present invention has been made in order to solve the above-described problems occurring in the prior art, and it is an object of the present invention to provide a miRNA which may be used as a biomarker for predicting the prognosis of head and neck cancer. Another object of the present invention is to determine the expression level of the miRNA in head and neck disease and to more rapidly and accurately identify a subtype of head and neck cancer based on the expression of the miRNA.

Still another object of the present invention is to predict the prognosis of a subtype of head and neck cancer and to provide a kit for identifying a subtype of head and neck disease or determining the prognosis of head and neck cancer disease.

However, objects which are to be achieved by the present invention are not limited to the above-mentioned objects, and other objects of the present invention will be clearly understood by those skilled in the art from the following description.

### Technical Solution

Hereinafter, various embodiments described herein will be described with reference to figures. In the following description, numerous specific details are set forth, such as specific configurations, compositions, and processes, etc., in order to provide a thorough understanding of the present invention. However, certain embodiments may be practiced without one or more of these specific details, or in combination with other known methods and configurations. In other instances, known processes and preparation techniques have not been described in particular detail in order to not unnecessarily obscure the present invention. Reference throughout this specification to "one embodiment" or "an embodiment" means that a particular feature, configuration, composition, or characteristic described in connection with the embodiment is included in at least one embodiment of the present invention. Thus, the appearances of the phrase "in one embodiment" or "an embodiment" in various places throughout this specification are not necessarily referring to the same embodiment of the present invention. Additionally, the particular features, configurations, compositions, or characteristics may be combined in any suitable manner in one or more embodiments.

Unless otherwise stated in the specification, all the scientific and technical terms used in the specification have the same meanings as commonly understood by those skilled in the technical field to which the present invention.

Hereinafter, each step of the present invention will be described in detail.

In one embodiment of the present invention, "head and neck cancer" specifically means head and neck squamous cell carcinoma, and the term "head and neck squamous cell carcinoma" or "HNSCC" includes, but is not limited to, cancers of organs, including mouth, lips, nasal cavity, paranasal sinuses, pharynx, larynx, nasopharynx, throat and the like. In the present invention, there are named two phenotypes (stromal and tumor subtypes) of head and neck squamous cell carcinoma according to their respective correlations with functional target gene ontology, and these are associated with different stages of epithelial cell differentiation.

In one embodiment of the present invention, "miRNA" is a single-stranded RNA molecule consisting of 17 to 25 nucleotides, and is a novel substance that controls gene expression in eukaryotes by binding to the 3'-UTR of mRNA. Regarding the production of miRNA, pre-miRNA having a stem-loop structure is made by Drosha (RNaseIII type enzyme), migrates to the cytoplasm, and cut by a dicer, thus making miRNA. The miRNA made as described above is involved in development, cell proliferation and death, lipid metabolism, tumor formation and the like by controlling expression of a target protein. In addition, it plays an important role in the regulation of various physiological activities including immune responses in host cells. In recent years, miRNAs have been deeply studied as new types of diagnostic biomarkers in various fields, including cancer, heart diseases, pregnancy, diabetes, mental illnesses and the like.

In the present invention, 41 reliable miRNAs were selected using interclass correlation coefficients (ICCs). The miRNAs regulate the development, growth and differentiation of epithelial cells, and are used to identify the subtype of HNSCC based on their expression patterns in each epithelial cell tissue and stromal tissue. The 41 miRNAs that are used as biomarkers of head and neck cancer are divided into a first group and a second group. The miRNA of the first group means one selected from the group consisting of miR-206, miR-127-5p, miR-432-5p, miR-409-5p, miR-145-3p, miR-497-5p, miR-195-5p, miR-150-5p, miR-140-3p, miR-214-3p, miR-125b-5p and miR-99a-5p, and the miRNA of the second group means one selected from the group consisting of miR-203a, miR-200a-5p, miR-200a-3p, miR-200b-5p, miR-200b-3p, miR-200c-3p, miR-141-3p, miR-205-5p, miR-183-5p, miR-106b-3p, miR-93-5p, miR-17-3p, miR-17-5p, miR-20a-5p, miR-92a-3p, miR-744-5p, miR-324-3p, miR-193a-5p, miR-149-5p, miR-1307-3p, miR-107, miR-103a-3p, miR-221-3p, miR222, miR-532-5p, miR-24-3p, miR-27b-5p, miR-27b-3p and miR-23b-3p.

In one embodiment of the present invention, "biomarker" refers to a marker that can distinguish between normal and pathological conditions or that can predict therapeutic responses and makes objective measurement possible. The expression levels of the miRNAs of a first group and a second group increase or decrease depending on head and neck cancer subtypes. Thus, head and neck cancer subtypes can be significantly diagnosed by measuring the expression levels of the miRNAs in a biological sample from a subject to be analyzed.

As used herein, the term "prognosis" includes determination as to whether a subject is presently affected by a specific disease or disorder, identification of the responsiveness of a subject to therapy, or use of therametrics (e.g., monitoring a subject's condition to provide information as to the efficacy of therapy).

In one embodiment of the present invention, "measuring the expression level of miRNA" is a process of determining the presence or expression level of miRNA of disease marker genes in a biological sample in order to identify head and neck cancer subtypes, and may be performed by measuring the level of miRNA. Analysis methods for this measurement include, but are not limited to, reverse transcription polymerase chain reaction (RT-PCR), competitive RT-PCR, real-time RT-PCR, RNase protection assay (RPA), Northern blotting, DNA chip assay or the like.

An agent for measuring the miRNA expression level of the gene means a molecule which may be used to detect the marker by determining the expression level of a miRNA whose expression in the cell of interest increases or decreases. The agent may preferably include, but is not limited to, a primer or a probe, which binds specifically to the gene. Based on the polynucleotide sequences of the miRNAs, those skilled in the art may design either a primer for specifically amplifying a specific region of these genes or a probe.

"Primer" in the present invention means a short nucleic acid sequence containing a free 3' hydroxyl group, which may form base pairs with a complementary template and functions as the starting point to copy the template strand. The primer may initiate DNA synthesis in the presence of four different nucleoside triphosphates and an agent for polymerization (i.e., DNA polymerase or reverse transcriptase) in an appropriate buffer and at a suitable temperature. In the present invention, disease may be diagnosed by performing PCR amplification using sense and antisense primers for a miRNA polynucleotide and determining whether a desired product would be produced. PCR conditions and the lengths of sense and antisense primers may be modified based on those known in the art.

The primer or probe used in the present invention may be chemically synthesized using a phosphoramidite solid support method or other widely known methods. These nucleic acid sequences may also be modified using many means known in the art. Non-limiting examples of such modifications include methylation, capping, replacement of one or more native nucleotides with analogues thereof, and inter-nucleotide modifications, for example, modifications to uncharged conjugates (e.g., methyl phosphonate, phosphotriester, phosphoroamidate, carbamate, etc.) or charged conjugates (e.g., phosphorothioate, phosphorodithioate, etc.).

In one embodiment of the present invention, "kit" means a set of compositions and accessories required for a specific purpose. For the purpose of the present invention, the kit of the present disclosure is for diagnosing a subtype of head and neck cancer disease or determining the prognosis of head and neck cancer disease. The kit of the present disclosure may include a primer for identifying a subtype of head and neck cancer or determining the prognosis of head and neck cancer disease, a probe, optionally an antibody that recognizes a peptide, or an antibody that recognizes a specific peptide whose expression specifically changes when affected by head and neck cancer disease, as well as one or more compositions, solutions or devices suitable for analysis.

In a first aspect of the present invention, there is provided a method for identifying a subtype of head and neck cancer, the method including the steps of: measuring the expression levels of miRNAs of a first group and a second group in a biological sample isolated from a subject; and identifying the subtype of head and neck cancer based on the expression patterns of the first group and the second group, wherein the first group is any one or more selected from the group consisting of miR-206, miR-127-5p, miR-432-5p, miR-409-5p, miR-145-3p, miR-497-5p, miR-195-5p, miR-150-5p, miR-140-3p, miR-214-3p, miR-125b-5p and miR-99a-5p, and the second group is any one or more selected from the group consisting of miR-203a, miR-200a-5p, miR-200a-3p, miR-200b-5p, miR-200b-3p, miR-200c-3p, miR-141-3p, miR-205-5p, miR-183-5p, miR-106b-3p, miR-93-5p, miR-17-3p, miR-17-5p, miR-20a-5p, miR-92a-3p, miR-744-5p, miR-324-3p, miR-193a-5p, miR-149-5p, miR-1307-3p, miR-107, miR-103a-3p, miR-221-3p, miR222, miR-532-5p, miR-24-3p, miR-27b-5p, miR-27b-3p and miR-23b-3p, wherein (i) when the miRNA of the first group is up-regulated, the subtype of head and neck cancer is identified as a stromal subtype, (ii) when the miRNA of the second group is down-regulated, the subtype of head and neck cancer is identified as a stromal subtype, (iii) when the miRNA of the first group is down-regulated, the subtype of head and neck cancer is identified as a tumor subtype, or (iv) when the miRNA of the second group is up-regulated, the subtype of head and neck cancer is identified as a tumor subtype. In one embodiment of the first aspect, the biological sample in the step is a head and neck tissue. In one embodiment of the first aspect, the expression levels of the miRNAs in the step are measured by performing a microarray or RNA sequencing method. In one embodiment of the first aspect, the head and neck cancer is head and neck squamous cell carcinoma (HNSCC).

Also disclosed is a kit for identifying a subtype of head and neck cancer, the kit including a primer or probe complementary to a miRNA obtained from a subject, wherein the miRNA is selected from the group consisting of a first group and a second group, wherein the first group is any one or more selected from the group consisting of miR-206, miR-127-5p, miR-432-5p, miR-409-5p, miR-145-3p, miR-497-5p, miR-195-5p, miR-150-5p, miR-140-3p, miR-214-3p, miR-125b-5p and miR-99a-5p, and the second group is any one or more selected from the group consisting of miR-203a, miR-200a-5p, miR-200a-3p, miR-200b-5p, miR-200b-3p, miR-200c-3p, miR-141-3p, miR-205-5p, miR-183-5p, miR-106b-3p, miR-93-5p, miR-17-3p, miR-17-5p, miR-20a-5p, miR-92a-3p, miR-744-5p, miR-324-3p, miR-193a-5p, miR-149-5p, miR-1307-3p, miR-107, miR-103a-3p, miR-221-3p, miR222, miR-532-5p, miR-24-3p, miR-27b-5p, miR-27b-3p and miR-23b-3p. When the miRNA of the first group is up-regulated, the subtype of head and neck cancer is identified as a stromal subtype. When the miRNA of the second group is down-regulated, the subtype of head and neck cancer is identified as a stromal subtype. When the miRNA of the first group is down-regulated, the subtype of head and neck cancer is identified as a tumor subtype. When the miRNA of the second group is up-regulated, the subtype of head and neck cancer is identified as a tumor subtype.

In a second aspect of the present invention, there is provided a method for providing information for predicting the prognosis of a subtype of head and neck cancer, the method including the step of: measuring the expression levels of miRNAs of a first group and a second group in a biological sample isolated from a subject; and identifying the subtype of head and neck cancer based on the expression patterns of the first group and the second group, wherein the first group is any one or more selected from the group consisting of miR-206, miR-127-5p, miR-432-5p, miR-409-5p, miR-145-3p, miR-497-5p, miR-195-5p, miR-150-5p, miR-140-3p, miR-214-3p, miR-125b-5p and miR-99a-5p, and the second group is any one or more selected from the group consisting of miR-203a, miR-200a-5p, miR-200a-3p, miR-200b-5p, miR-200b-3p, miR-200c-3p, miR-141-3p, miR-205-5p, miR-183-5p, miR-106b-3p, miR-93-5p, miR-17-3p, miR-17-5p, miR-20a-5p, miR-92a-3p, miR-744-5p, miR-324-3p, miR-193a-5p, miR-149-5p, miR-1307-3p, miR-107, miR-103a-3p, miR-221-3p, miR222, miR-532-5p, miR-24-3p, miR-27b-5p, miR-27b-3p and miR-23b-3p.

When the miRNA of the first group is up-regulated, the subtype of head and neck cancer is identified as a stromal subtype and may be determined to have poorer prognosis than a tumor subtype. In one embodiment, prognosis when one or more miRNAs selected from the group consisting of miR-127, miR-409, miR-432 and miR-27b-5p are expressed is determined. When the miRNA of the second group is down-regulated, the subtype of head and neck cancer is identified as a stromal subtype and may be determined to have poorer prognosis than a tumor subtype. When the miRNA of the first group is down-regulated, the subtype of head and neck cancer is identified as a tumor subtype and may be determined to have better prognosis than a stromal subtype. When the miRNA of the second group is up-regulated, the subtype of head and neck cancer is identified as a tumor subtype and may be determined to have better prognosis than a stromal subtype.

Also disclosed is provided a kit for predicting the prognosis of head and neck cancer, the kit including a primer or probe complementary to a miRNA obtained from a subject, wherein the miRNA is selected from the group consisting of a first group and a second group, wherein the first group is any one or more selected from the group consisting of miR-206, miR-127-5p, miR-432-5p, miR-409-5p, miR-145-3p, miR-497-5p, miR-195-5p, miR-150-5p, miR-140-3p, miR-214-3p, miR-125b-5p and miR-99a-5p, and the second group is any one or more selected from the group consisting of miR-203a, miR-200a-5p, miR-200a-3p, miR-200b-5p, miR-200b-3p, miR-200c-3p, miR-141-3p, miR-205-5p, miR-183-5p, miR-106b-3p, miR-93-5p, miR-17-3p, miR-17-5p, miR-20a-5p, miR-92a-3p, miR-744-5p, miR-324-3p, miR-193a-5p, miR-149-5p, miR-1307-3p, miR-107, miR-103a-3p, miR-221-3p, miR222, miR-532-5p, miR-24-3p, miR-27b-5p, miR-27b-3p and miR-23b-3p. When the miRNA of the first group is up-regulated, the subtype of head and neck cancer is identified as a stromal subtype and determined to have poorer prognosis than a tumor subtype. Prognosis when one or more miRNAs selected from the group consisting of miR-127, miR-409, miR-432 and miR-27b-5p are expressed may be determined. When the miRNA of the second group is down-regulated, the subtype of head and neck cancer is identified as a stromal subtype and may be determined to have poorer prognosis than a tumor subtype. When the miRNA of the first group is down-regulated, the subtype of head and neck cancer is identified as a tumor subtype and may be determined to have better prognosis than a stromal subtype. When the miRNA of the second group is up-regulated, the subtype of head and neck cancer is identified as a tumor subtype and may be determined to have better prognosis than a stromal subtype.

In a third aspect of the present invention, there is provided a method for selecting a miRNA, including: a first step of obtaining miRNA expression data from a sample with head and neck cancer; a second step of obtaining miRNA expression data from a head and neck cancer-related database; and a step of selecting an miRNA from the top 15% of the first step and the second step, which are correlated, wherein the second step is capable of identifying a subtype of head and neck cancer whose expression data are averaged and have a mean absolute deviation of 75% or less. The miRNA is preferably selected from the group consisting of a first group and a second group, wherein the first group is any one or more selected from the group consisting of miR-206, miR-127-5p, miR-432-5p, miR-409-5p, miR-145-3p, miR-497-5p, miR-195-5p, miR-150-5p, miR-140-3p, miR-214-3p, miR-125b-5p and miR-99a-5p, and the second group is any one or more selected from the group consisting of miR-203a, miR-200a-5p, miR-200a-3p, miR-200b-5p, miR-200b-3p, miR-200c-3p, miR-141-3p, miR-205-5p, miR-183-5p, miR-106b-3p, miR-93-5p, miR-17-3p, miR-17-5p, miR-20a-5p, miR-92a-3p, miR-744-5p, miR-324-3p, miR-193a-5p, miR-149-5p, miR-1307-3p, miR-107, miR-103a-3p, miR-221-3p, miR222, miR-532-5p, miR-24-3p, miR-27b-5p, miR-27b-3p and miR-23b-3p, wherein (i) when the miRNA of the first group is up-regulated, the subtype of head and neck cancer is identified as a stromal subtype, (ii) when the miRNA of the second group is down-regulated, the subtype of head and neck cancer is identified as a stromal subtype, (iii) when the miRNA of the first group is down-regulated, the subtype of head and neck cancer is identified as a tumor subtype, or (iv) when the miRNA of the second group is up-regulated, the subtype of head and neck cancer is identified as a tumor subtype.

### Advantageous Effects

Head and neck cancer is one of the most frequently encountered cancers with low survival rates. In particular, head and neck squamous cell carcinoma is classified as a malignant tumor. Thus, the use of biomarkers for predicting the prognosis of head and neck cancer has been demanded. In order to solve the above-described problems occurring in the prior art, the present invention can more rapidly and accurately identify a subtype of head and neck cancer based on the expression patterns of miRNA, and provides a biomarker which can determine the prognosis of head and neck cancer and which is expected to be effectively used for the prediction of prognosis of head and neck cancer.

### Description of Drawings

FIG. 1 is a flow chart showing selecting 41 miRNAs based on interclass correlation coefficients according to an example of the present invention.
FIG. 2 shows time-dependent changes in miRNA expression profiles in normal airway epithelial development models according to an example of the present invention.
FIG. 3 is a heat map showing the expression of 41 miRNAs according to an example of the present invention.
FIG. 4 is a heat map showing the relationship between the expression levels of 41 miRNAs and airway primary cell signal oncogenic pathways according to an example of the present invention.
FIG. 5 is a heat map showing 41 miRNAs and gene ontology functional categories according to an example of the present invention.
FIG. 6 shows the expressions of 41 miRNAs in UNC and TCGA samples for a HNSCC subtype according to an example of the present invention.
FIG. 7 is a heat map showing the expression of mRNA according to an example of the present invention.
FIG. 8 shows a difference in survival between tumor subtype and stromal subtype patients according to an example of the present invention.
FIG. 9 shows a difference in survival between HPV-infected patients and non-HPV-infected patients in a tumor subtype and a stromal subtype according to an example of the present invention.

### Mode for Invention

Hereinafter, the present invention will be described in more detail with reference to examples. However, it will be obvious to those skilled in the art that these examples are only for explaining the present invention in more detail and that the scope of the present invention according to the gist of the present invention is not limited by these examples, but rather the scope of the present invention is defined in the appended claims.

### Example 1: Preparation and Analysis of Head and Neck Tumor Samples

### Example 1-1. Preparation of Head and Neck Tumor Samples

Patient tissue samples were obtained through protocols approved by the Institutional Review Boards of the University of North Carolina (UNC). The discovery cohort was obtained from the UNC. Patients included in the methods of the present invention should be histologically confirmed HNSCC patients, and the medical records should be rigorously reviewed to obtain clinical and pathological data. To confirm HNSCC, cases of tumors located in the primary site of the oral cavity, oropharynx, hypopharynx and larynx were included, and cases where confirmation was difficult were excluded. Freshly frozen HNSCC samples from untreated patients were obtained through surgery from the UNC Tissue Procurement Core Facility or UNC Hospital. Five normal tissues and five tonsil epithelium samples from five pediatric patients who underwent general tonsillectomy were subjected to miRNA profiling using normal tissue.

### Example 1-2. RNA Isolation, Labeling, Microarray Hybridization and UNC HNSCC Sample Analysis

Total RNA containing small RNAs was extracted from surgical samples using TRIzol (Invitrogen). The RNA quantity was determined using a NanoDrop ND-1000 spectrometer (NanoDrop Technologies, Wilmington, DE), and the RNA integrity was assessed on an Agilent 2100 Bioanalyzer (Agilent Technologies, Palo Alto, CA). 1 µg of the total RNA was labeled using a FlashTaq Biotin RNA labeling kit (Genisphere Inc. Hatfield, PA). Namely, a poly (A) tail was first added to the RNA, and then a biotinylated signal molecule was bound to the target RNA sample. Thereafter, the biotin-labeled RNA was mixed with a hybridization mix and hybridized to an affymetrix GeneChip miRNA array overnight at 48°C. After the hybridization, the array was removed from the oven, washed, and then scanned with GeneChip Scanner 3000.

### Example 2: Measurement of miRNA Expression Levels

### Example 2-1. miRNA Expression and Sequence Data

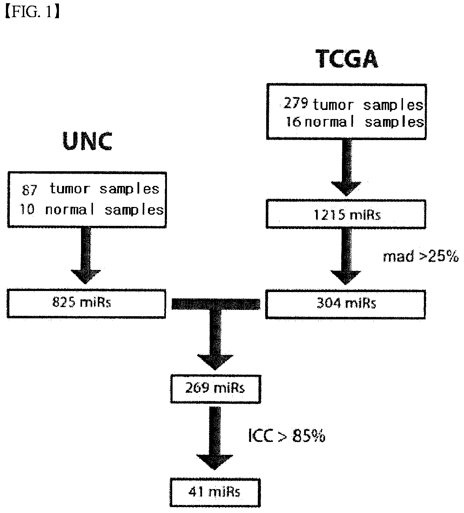

After miRNA expression profiling of the UNC sample was performed, quality control procedures were applied to microarray probe-level intensity files, and the API package was used to process CEL files. A total of 87 tumor samples and 10 normal samples remained after removal of low-quality arrays and replicated arrays. Probes were selected to produce expression values for 825 miRNAs. Permission to access TCGA data was obtained from the Data Access Committee for the NCBI dbGAP (National Center for Biotechnology Information Genotypes and Phenotypes Database) at the National Institutes of Health. miRNA expression data processed (level 3) from the validation cohort were downloaded from the TCGA portal. Expression data for 1215 human miRNAs were averaged, and the standard deviation was normalized per array. The data with low variability (mean absolute deviation or MAD < 75 percentile) in expression levels were filtered and the resulting 304 remaining miRNAs were log2-transformed. In order to technically validate differentially expressed miRNAs produced from two different platforms and cohorts, 269 overlapping miRNAs were used in analysis. Then, to confirm miRNAs whose reproducibility was measured, interclass correlation coefficients were used for calculation. A total of 41 miRNAs in the top 15% of ICCs were considered reliable and used for further analysis. FIG. 1 shows a flow chart showing the 41 miRNAs selected based on the ICCs.

### Example 2-2. Consensus Clustering of miRNA Expression Profiles and Validation

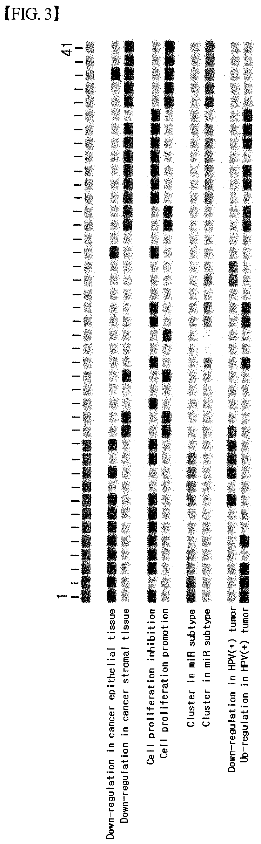

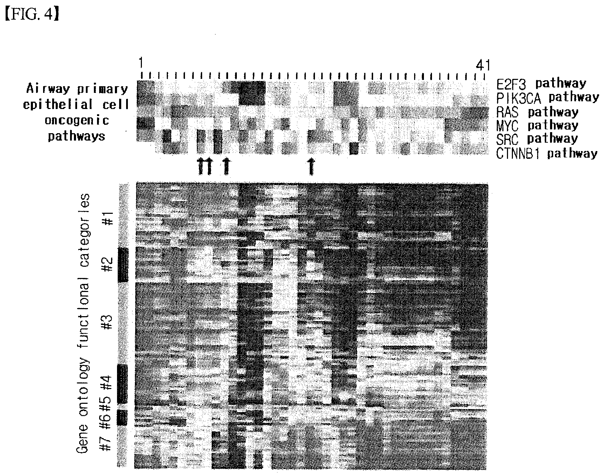

In the discovery and validation cohorts, miRNA subtypes were determined using the Consensus Cluster Plus R package. Consensus clustering was performed using average linkage and a hierarchical clustering method with 1-Pearson's correlation coefficient as the distance metric. Using graphic output from the Consensus Cluster Plus, the optimal number of miRNA subgroups was determined. To determine the statistical significance of different expressions shown in the miRNA clusters, SigClust was applied using a set of the above-described 41 miRNAs. Through calculation of the average expression value of each miRNA in all samples in given cohorts with suitable subtype labels, the centroid for each miRNA subtype in the UNC and TCGA cohorts was produced. The predicted class label was produced by minimizing the correlation-based distance (1-Pearson correlation coefficient) for each centroid. In addition, to confirm the reproducibility of miRNA subtypes, robust miRNA clusters were applied to independently published HNSCC data, Avissar data, and bladder cancer data in lung SCC, uterine SCC and TCGA. The 41 miRNAs from 87 UNC and 279 TCGA HNASCC patients were clustered according to their expression patterns into a first group and a second group, and FIG. 4 shows the expression level of miRNA according to each HNSCC subtype. The miRNAs of the first group were down-regulated in epithelial cell tissue, and the miRNAs of the second group were down-regulated in stromal tissue (FIG. 3).

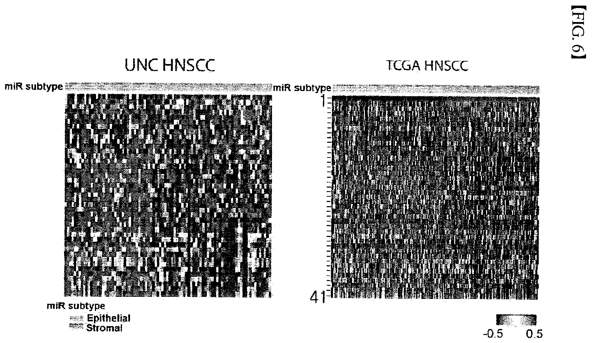

Names corresponding to Nos. 1 to 41 in FIGS. 3, 4 and 6 are shown in Table 1 below.

**[Table 1]**

| No. | Name | No. | Name | No. | Name |
|---|---|---|---|---|---|
| 1 | miR-99a-5p | 15 | miR-193a-5p | 29 | miR-93-5p |
| 2 | miR-125b-5p | 16 | miR-744-5p | 30 | miR-200c-3p |
| 3 | miR-497-5p | 17 | miR-532-5p | 31 | miR-200a-5p |
| 4 | miR-195-5p | 18 | miR-23b-3p | 32 | miR-200b-5p |
| 5 | miR-150-5p | 19 | miR-107 | 33 | miR-141-3p |
| 6 | miR-140-3p | 20 | miR-103a-3p | 34 | miR-200b-3p |
| 7 | miR-145-3p | 21 | miR-27b-5p | 35 | miR-200a-3p |
| 8 | miR-127-5p | 22 | miR-27b-3p | 36 | miR-149-5p |
| 9 | miR-409-5p | 23 | miR-361-5p | 37 | miR-183-5p |
| 10 | miR-214-3p | 24 | miR-24-3p | 38 | miR-17-3p |
| 11 | miR-432-5p | 25 | miR-1307-3p | 39 | miR-92a-3p |
| 12 | miR-206 | 26 | miR-205-5p | 40 | miR-17-5p |
| 13 | miR-203a | 27 | miR-324-3p | 41 | miR-20a-5p |
| 14 | miR-221-3p | 28 | miR-106b-3p | | |

### Example 2-3. Differentially Expressed miRNAs and Genes

To confirm differentially expressed miRNAs (DEmiRs) in a tumor group (tumor versus normal group or HPV-positive versus HPV-negative HNSCC), package samR was used. Different expressions were assessed using a two-class unpaired test, a random sheet and a false discovery rate (FDR) of 0.05 obtained by a 1,000-sequence. For differentially expressed mRNAs (DEG) between miRNA subtypes, samR was applied to a very variable gene set (MAD > 75 percentile) using a median false discovery rate (FDR) of 0.05. In HPV-positive HNSCC, 15 miRNAs were up-regulated and 4 miRNAs were down-regulated. The down-regulated miRNAs were all identified as the miRNAs of the first group.

### Example 2-4. miRNAs and Airway Primary Cell Signal Oncogenic Pathways

To assess the correlation between the expression levels of miRNAs and airway primary cell signal oncogenic pathway-related gene signatures, 6 cell signal pathway-related gene signatures (BILD-HRAS, BILD-SRC, BILD-MYC, BILD_CTNNB1, BILD-E2F3, BILD-PI3K) were analyzed using MSigDB representative of genes up-regulated in RAS, SRC, CTNNB1, MYC, E2F3 and PIK3CA pathways. Pearson's correlation coefficients between the expressions of individual genes in each gene set and individual miRNAs were calculated. Thereafter, the mean correlation values in each gene set were transformed into Z-scores and shown in a heat map (FIG. 4). Higher Z-scores indicate that the miRNA expression levels are correlated with gene expression in the HNSCC sample. Lower Z-scores indicate that there is a negative correlation between the expression of miRNAs and the expression of the gene set in the HNSCC sample.

### Example 2-5. miRNA-mRNA Connection and Integrated Analysis

In order to confirm the co-expression network between miRNAs and mRNAs, the expression profiles of 41 miRNAs and 20,500 genes were individually prepared from 279 TCGA HNSCC samples in which the miRNA and mRNA expression data were valid. Pearson's correlation coefficients were calculated for all possible combinations of items between the miRNAs and the genes. P-values were also produced and adjusted by the Benjamini and Hochberg correction (q-values). The exception of the negative correlation between miRNA and gene pair was determined to be a q-value of 0.01. Using the exception, 72,039 significantly correlated miRNA-vs.-gene pairs were obtained. Thereafter, the sequence complementarity approach was used to determine the direct interaction between individual miRNAs and mRNA targets using a predicted target list determined through at least two algorithms of targetScan, miRanda, miRWalk, PICTAR4, PICTAR5 and miRDB. Then, DAVID was used to find a KEGG pathway showing enrichment for genes overlapping between DEG for a miRNA-based subtype and a predicted target gene in each subtype. The gene ontology (GO) terms in the analyzed set of target genes are statistically most enriched. For extended functional correlation analysis, a gene cluster enrichment analysis (PAGE) approach employing a parametric method for confirming a biological subject that significantly changed in the expression data set was used. Gene sets showing GO categories was downloaded from MSigDB. Using Z-score for each GO category with significant enrichment (FDR<0.01), a matrix was constructed for 1,022 GO categories and 41 miRNAs (FIGS. 3 to 5). Hierarchical clustering was performed on the Z-score matrix. In addition, an R package cut tree was used in a group in which 1,022 GO categories did fall within 7 functional categories according to correlation similarity.

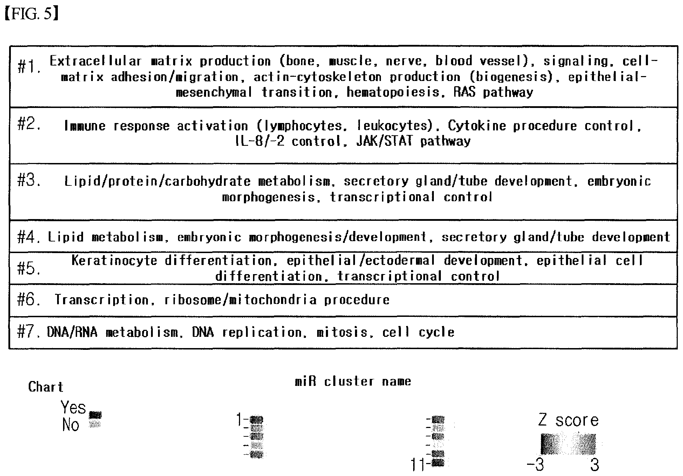

Names corresponding to Nos. 1 to 11 in the miR cluster names in FIG. 5 are shown in Table 2 below.

**[Table 2]**

| No. | Name | No. | Name |
|---|---|---|---|
| 1 | miR-99a/100-125b tricistrons | 7 | miR 106b-25 cluster (Chr7q22.1) |
| 2 | miR195/497 cluster | 8 | miR 200 family |
| 3 | RTL1 (Chr 14q32) | 9 | miR-103/107 family |
| 4 | miR 143/145 cluster | 10 | miR-23b/-27b cluster |
| 5 | miR 199/214 cluster | | (Chr 9q22.32) MET gene related |
| 6 | miR 17-92 cluster (Chr13q31.3) | 11 | miR-183/96/182 cluster |

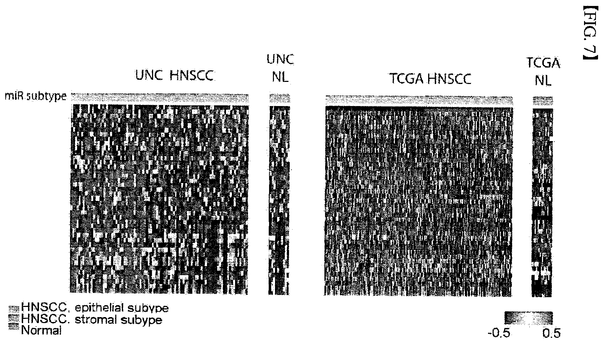

Using DAVID, it was shown that among 41 miRNAs and 6,701 genes (955 DEGs and 5,266 negatively correlated genes), 502 mRNAs were predicted targets and controlled by the 41 miRNAs. Through functional analysis of the 501 mRNAs, it can be seen that the tumor subtype was dominated by the miRNAs down-regulated in epithelial cancer tissue, and that up-regulation of epithelial cell markers (ELF, CSTA and SOX2) and transcription and protein migration markers (FOX1 and CPLX3) was enriched. In addition, in the stromal subtype dominated by the miRNAs down-regulated in cancer stromal tissue, it can be seen that up-regulation of soft tissue markers was enriched (FIG. 7). The soft tissue markers are as follows: muscle development (MYOM1 and MYF5), cell adhesion (CDH11, TGFBI, FN1 and COL5A1), EMT (MMP2, ZEB2, TWIST1, KLK5 and MET), blood vessel formation (VEGFC, VEGFR, PDGFA, CAV1 and EDN3), cell proliferation (RUNX2 and IGF1) and immune markers (TLR4, PDCD1, CXCL12 and CD8).

### Example 2-6. Survival Analysis

Coded patient survival data were extracted from the TCGA clinical information file. A Cox proportional hazards model was used to estimate association between survival time and the expression level of each miRNA. Thereafter, the P-value was adjusted using the Benjamini and Hochberg correction. To access the independent prognostic value of the subtype of each miRNA, multivariate Cox proportional hazards analyses were performed.

In the determination of prognosis, miR-127, miR-209, miR-432 and miR-27b-5p indicated by the arrows in FIG. 4 were shown to be statistically important prognosis prediction factors. High expressions of miR-127, miR-409 and miR-432, which are highly associated with the activities of RAS MYC, SRC and CTNNB 1 oncogenic pathways, indicated low prognosis. However, it can be seen that the expression of miR-27b-5p showed good prognosis.

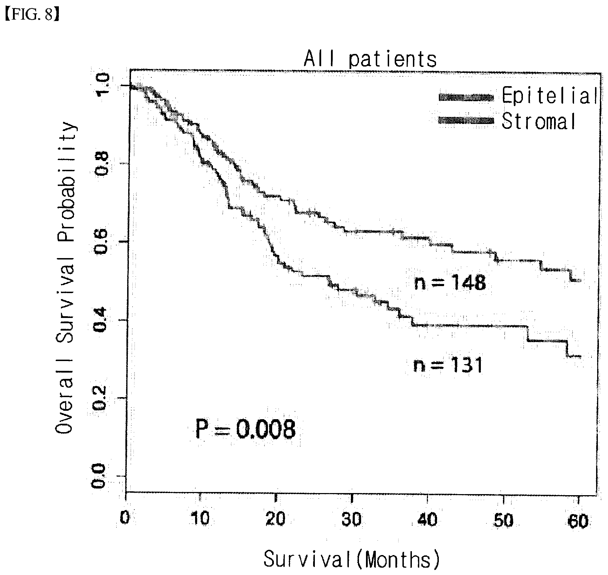

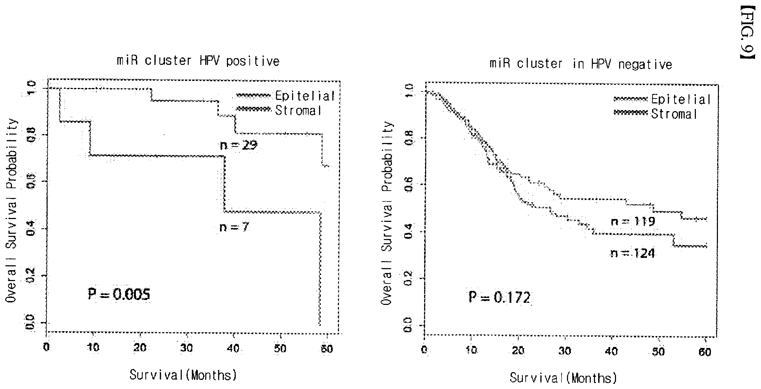

From FIGS. 8 and 9, it can be seen that survival rate is generally lower in the stromal subtype patient group than in the tumor subtype group (FIG. 8). In addition, HPV-infected patients showed a significant difference in the patient survival rate between HNSCC subtypes, whereas non-HPV-infected patients showed an insignificant difference between the subtypes (FIG. 9).

### Example 2-7. miRNA Profiles and Normal Airway Epithelial Development Models

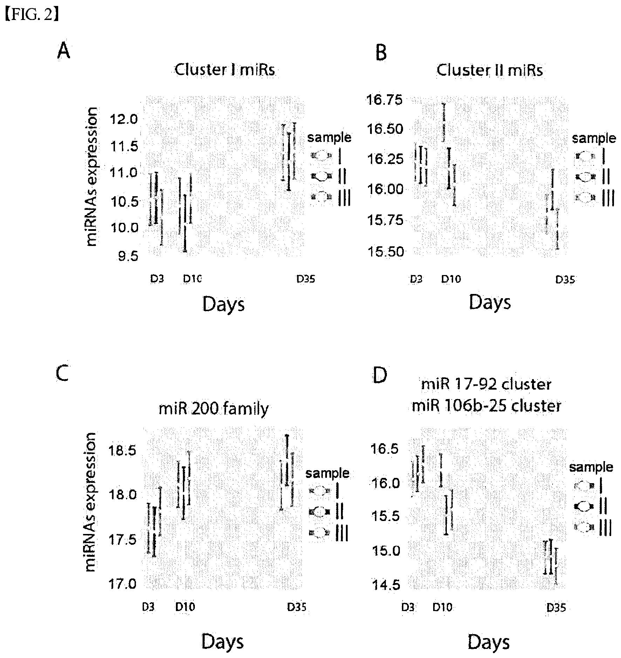

For a time-course study of miRNA profile changes during human bronchial epithelial (hBE) differentiation, normal hBE cells were grown on plates and collected on day 3, day 10 and day 35. RNA was extracted and a small RNA library was prepared. After miRNA expression profiling was performed, paired t-statistic was used to compare the changes in miRNA expression on day 3 and day 5. The changes in miRNA profiling at different time points were assessed in normal airway epithelial development models, and as a result, it was shown that the mean expression of miRNAs of the first group was initially low, and then increased as airway epithelial development progressed, whereas the expression of miRNAs of the second group was initially high, and then gradually decreased (FIG. 2).

## Claims

1. A method for identifying a subtype of head and neck cancer, comprising the steps of:
measuring expression levels of miRNAs of a first group and a second group in a biological sample isolated from a subject; and
identifying the subtype of head and neck cancer based on expression patterns of the first group and the second group,
wherein the first group is any one or more selected from the group consisting of miR-206, miR-127-5p, miR-432-5p, miR-409-5p, miR-145-3p, miR-497-5p, miR-195-5p, miR-150-5p, miR-140-3p, miR-214-3p, miR-125b-5p and miR-99a-5p, and
the second group is any one or more selected from the group consisting of miR-203a, miR-200a-5p, miR-200a-3p, miR-200b-5p, miR-200b-3p, miR-200c-3p, miR-141-3p, miR-205-5p, miR-183-5p, miR-106b-3p, miR-93-5p, miR-17-3p, miR-17-5p, miR-20a-5p, miR-92a-3p, miR-744-5p, miR-324-3p, miR-193a-5p, miR-149-5p, miR-1307-3p, miR-107, miR-103a-3p, miR-221-3p, miR222, miR-532-5p, miR-24-3p, miR-27b-5p, miR-27b-3p and miR-23b-3p,
wherein
(i) when the miRNA of the first group is up-regulated, the subtype of head and neck cancer is identified as a stromal subtype,
(ii) when the miRNA of the second group is down-regulated, the subtype of head and neck cancer is identified as a stromal subtype,
(iii) when the miRNA of the first group is down-regulated, the subtype of head and neck cancer is identified as a tumor subtype, or
(iv) when the miRNA of the second group is up-regulated, the subtype of head and neck cancer is identified as a tumor subtype.

2. The method of claim 1, wherein the biological sample in the step is a head and neck tissue.

3. The method of claim 1, wherein the expression levels of the miRNAs in the step are measured by performing a microarray or RNA sequencing method.

4. The method of claim 1, wherein the head and neck cancer is head and neck squamous cell carcinoma (HNSCC).

5. A method for providing information for predicting the prognosis of a subtype of head and neck cancer, the method comprising the steps of the method of claim 1, wherein the different subtypes are associated with different prognosis.

6. The method of claim 5 , wherein a stromal subtype is determined to have poorer prognosis than a tumor subtype.

7. The method of claim 6, wherein prognosis when one or more miRNAs selected from the group consisting of miR-127, miR-409, miR-432 and miR-27b-5p are expressed is determined.

8. The method of claim 5, wherein a tumor subtype is determined to have better prognosis than a stromal subtype.

9. A computer implemented method for selecting a miRNA as subtype indicator for head and neck cancer, comprsing:
a first step of obtaining miRNA expression data from a sample with head and neck cancer;
a second step of obtaining miRNA expression data from a head and neck cancer-related database; and
a step of selecting a miRNA from the top 15% of those with correlating expression levels between expression levels of miRNAs from the first step and the second step, wherein the database of the second step containing expression data capable of identifying a subset of head and neck cancer **characterised by** the expression data which are averaged and have mean absolute deviation of 75% or less,
wherein the miRNA is preferably selected from the group consisting of a first group and a second group,
wherein the first group is any one or more selected from the group consisting of miR-206, miR-127-5p, miR-432-5p, miR-409-5p, miR-145-3p, miR-497-5p, miR-195-5p, miR-150-5p, miR-140-3p, miR-214-3p, miR-125b-5p and miR-99a-5p, and
the second group is any one or more selected from the group consisting of miR-203a, miR-200a-5p, miR-200a-3p, miR-200b-5p, miR-200b-3p, miR-200c-3p, miR-141-3p, miR-205-5p, miR-183-5p, miR-106b-3p, miR-93-5p, miR-17-3p, miR-17-5p, miR-20a-5p, miR-92a-3p, miR-744-5p, miR-324-3p, miR-193a-5p, miR-149-5p, miR-1307-3p, miR-107, miR-103a-3p, miR-221-3p, miR222, miR-532-5p, miR-24-3p, miR-27b-5p, miR-27b-3p and miR-23b-3p,
wherein
(i) when the miRNA of the first group is up-regulated, the subtype of head and neck cancer is identified as a stromal subtype,
(ii) when the miRNA of the second group is down-regulated, the subtype of head and neck cancer is identified as a stromal subtype,
(iii) when the miRNA of the first group is down-regulated, the subtype of head and neck cancer is identified as a tumor subtype, or
(iv) when the miRNA of the second group is up-regulated, the subtype of head and neck cancer is identified as a tumor subtype.

## Patentansprüche

1. Verfahren zum Identifizieren eines Subtyps von Kopf-und-Hals-Krebs, umfassend die Schritte:
Messen von Expressionsspiegeln von miRNAs einer ersten Gruppe und einer zweiten Gruppe in einer biologischen, aus einem Lebewesen isolierten Probe; und
Identifizieren des Subtyps von Kopf-und-Hals-Krebs basierend auf Expressionsmustern der ersten Gruppe und der zweiten Gruppe,
wobei die erste Gruppe eine jegliche oder mehrere ist, die aus der Gruppe ausgewählt ist/sind, die aus miR-206, miR-127-5p, miR-432-5p, miR-409-5p, miR-145-3p, miR-497-5p, miR-195-5p, miR-150-5p, miR-140-3p, miR-214-3p, miR-125b-5p und miR-99a-5p besteht, und
die zweite Gruppe eine jegliche oder mehrere ist, die aus der Gruppe ausgewählt ist/sind, die aus miR-203a, miR-200a-5p, miR-200a-3p, miR-200b-5p, miR-200b-3p, miR-200c-3p, miR-141-3p, miR-205-5p, miR-183-5p, miR-106b-3p, miR-93-5p, miR-17-3p, miR-17-5p, miR-20a-5p, miR-92a-3p, miR-744-5p, miR-324-3p, miR-193a-5p, miR-149-5p, miR-1307-3p, miR-107, miR-103a-3p, miR-221-3p, miR222, miR-532-5p, miR-24-3p, miR-27b-5p, miR-27b-3p und miR-23b-3p besteht,
wobei
(i) wenn die miRNA der ersten Gruppe hochreguliert ist, der Subtyp von Kopf-und-Hals-Krebs als ein Stroma-Subtyp identifiziert wird,
(ii) wenn die miRNA der zweiten Gruppe herunterreguliert ist, der Subtyp von Kopf-und-Hals-Krebs als ein Stroma-Subtyp identifiziert wird,
(iii) wenn die miRNA der ersten Gruppe herunterreguliert ist, der Subtyp von Kopf-und-Hals-Krebs als ein Tumor-Subtyp identifiziert wird, oder
(iv) wenn die miRNA der zweiten Gruppe hochreguliert ist, der Subtyp von Kopf-und-Hals-Krebs als ein Tumor-Subtyp identifiziert wird.

2. Verfahren nach Anspruch 1, wobei die biologische Probe in dem Schritt ein Kopf-und-Hals-Gewebe ist.

3. Verfahren nach Anspruch 1, wobei die Expressionsspiegel der miRNAs in dem Schritt durch Durchfuhren eines Microarray- oder RNA-Sequenzierungsverfahrens gemessen werden.

4. Verfahren nach Anspruch 1, wobei der Kopf-und-Hals-Krebs Kopf-und-Hals-Plattenepithelkarzinom (HNSCC) ist.

5. Verfahren zum Bereitstellen von Information für eine Vorhersage der Prognose eines Subtyps von Kopf-und-Hals-Krebs, wobei das Verfahren die Schritte des Verfahrens von Anspruch 1 umfasst, wobei die unterschiedlichen Subtypen mit unterschiedlicher Prognose assoziiert sind.

6. Verfahren nach Anspruch 5, wobei bestimmt wird, dass ein Stroma-Subtyp eine schlechtere Prognose hat als ein Tumor-Subtyp.

7. Verfahren nach Anspruch 6, wobei eine Prognose, wenn eine oder mehrere miRNAs, ausgewählt aus der Gruppe, bestehend aus miR-127, miR-409, miR-432 und miR-27b-5p, exprimiert werden, bestimmt wird.

8. Verfahren nach Anspruch 5, wobei bestimmt wird, dass ein Tumor-Subtyp eine bessere Prognose hat als ein Stroma-Subtyp.

9. Computerimplementiertes Verfahren zum Auswählen einer miRNA als Subtyp-Indikator für Kopf-und-Hals-Krebs, umfassend:
einen ersten Schritt eines Erhaltens von miRNA-Expressionsdaten aus einer Probe mit Kopf-und-Hals-Krebs;
einen zweiten Schritt eines Erhaltens von miRNA-Expressionsdaten aus einer Datenbank, die mit Kopf-und-Hals-Krebs in Beziehung steht; und
einen Schritt eines Auswählens einer miRNA aus den oberen 15% von denjenigen mit korrelierenden Expressionsspiegeln zwischen Expressionsspiegeln von miRNAs aus dem ersten Schritt und dem zweiten Schritt, wobei die Datenbank des zweiten Schritts Expressionsdaten enthält, die fähig sind, eine Untergruppe von Kopf-und-Hals-Krebs, charakterisiert durch die Expressionsdaten, die gemittelt sind und eine mittlere absolute Abweichung von 75% oder
weniger aufweisen, zu identifizieren,
wobei die miRNA vorzugsweise aus der Gruppe ausgewählt ist, die aus einer ersten Gruppe und einer zweiten Gruppe besteht,
wobei die erste Gruppe eine jegliche oder mehrere ist, die aus der Gruppe ausgewählt ist/sind, die aus miR-206, miR-127-5p, miR-432-5p, miR-409-5p, miR-145-3p, miR-497-5p, miR-195-5p, miR-150-5p, miR-140-3p, miR-214-3p, miR-125b-5p und miR-99a-5p besteht, und die zweite Gruppe eine jegliche oder mehrere ist, die aus der Gruppe ausgewählt ist/sind, die aus miR-203a, miR-200a-5p, miR-200a-3p, miR-200b-5p, miR-200b-3p, miR-200c-3p, miR-141-3p, miR-205-5p, miR-183-5p, miR-106b-3p, miR-93-5p, miR-17-3p, miR-17-5p, miR-20a-5p, miR-92a-3p, miR-744-5p, miR-324-3p, miR-193a-5p, miR-149-5p, miR-1307-3p, miR-107, miR-103a-3p, miR-221-3p, miR222, miR-532-5p, miR-24-3p, miR-27b-5p, miR-27b-3p und miR-23b-3p besteht, wobei
(i) wenn die miRNA der ersten Gruppe hochreguliert ist, der Subtyp von Kopf-und-Hals-Krebs als ein Stroma-Subtyp identifiziert wird,
(ii) wenn die miRNA der zweiten Gruppe herunterreguliert ist, der Subtyp von Kopf-und-Hals-Krebs als ein Stroma-Subtyp identifiziert wird,
(iii) wenn die miRNA der ersten Gruppe herunterreguliert ist, der Subtyp von Kopf-und-Hals-Krebs als ein Tumor-Subtyp identifiziert wird, oder
(iv) wenn die miRNA der zweiten Gruppe hochreguliert ist, der Subtyp von Kopf-und-Hals-Krebs als ein Tumor-Subtyp identifiziert wird.

## Revendications

1. Procédé d'identification d'un sous-type de cancer de la tête et du cou, comprenant les étapes suivantes :
mesurer les niveaux d'expression des miARN d'un premier groupe et d'un second groupe dans un échantillon biologique isolé d'un sujet ; et
identifier le sous-type de cancer de la tête et du cou sur la base des profils d'expression du premier groupe et du second groupe,
dans lequel le premier groupe est un ou plusieurs éléments quelconques sélectionnés dans le groupe constitué par miR-206, miR-127-5p, miR-432-5p, miR-409-5p, miR-145-3p, miR-497-5p, miR-195-5p, miR-150-5p, miR-140-3p, miR-214-3p, miR-125b-5p et miR-99a-5p, et
le second groupe est un ou plusieurs éléments quelconques sélectionnés dans le groupe constitué par miR-203a, miR-200a-5p, miR-200a-3p, miR-200b-5p, miR-200b-3p, miR-200c-3p, miR-141-3p, miR-205-5p, miR-183-5p, miR-106b-3p, miR-93-5p, miR-17-3p, miR-17-5p, miR-20a-5p, miR-92a-3p, miR-744-5p, miR-324-3p, miR-193a-5p, miR-149-5p, miR-1307-3p, miR-107, miR-103a-3p, miR-221-3p, miR222, miR-532-5p, miR-24-3p, miR-27b-5p, miR-27b-3p et miR-23b-3p,
dans lequel
(i) lorsque le miARN du premier groupe est régulé positivement, le sous-type de cancer de la tête et du cou est identifié comme étant un sous-type stromal,
(ii) lorsque le miARN du second groupe est régulé négativement, le sous-type de cancer de la tête et du cou est identifié comme étant un sous-type stromal,
(iii) lorsque le miARN du premier groupe est régulé négativement, le sous-type de cancer de la tête et du cou est identifié comme un sous-type tumoral, ou
(iv) lorsque le miARN du second groupe est régulé positivement, le sous-type de cancer de la tête et du cou est identifié comme un sous-type tumoral.

2. Procédé selon la revendication 1, dans lequel l'échantillon biologique dans l'étape est un tissu de la tête et du cou.

3. Procédé selon la revendication 1, dans lequel les niveaux d'expression des miARN dans l'étape sont mesurés en réalisant un procédé de microréseau ou de séquençage d'ARN.

4. Procédé selon la revendication 1, dans lequel le cancer de la tête et du cou est un carcinome à cellules squameuses de la tête et du cou (HNSCC).

5. Procédé permettant de fournir des informations pour prédire le pronostic d'un sous-type de cancer de la tête et du cou, le procédé comprenant les étapes du procédé de la revendication 1, dans lequel les différents sous-types sont associés à un pronostic différent.

6. Procédé selon la revendication 5, dans lequel un sous-type stromal est déterminé comme ayant un pronostic plus mauvais qu'un sous-type tumoral.

7. Procédé selon la revendication 6, dans lequel on détermine le pronostic lorsqu'un ou plusieurs miARN sélectionnés dans le groupe constitué par miR-127, miR-409, miR-432 et miR-27b-5p sont exprimés.

8. Procédé selon la revendication 5, dans lequel un sous-type tumoral est déterminé comme ayant un meilleur pronostic qu'un sous-type stromal.

9. Procédé mis en œuvre par ordinateur pour sélectionner un miARN comme indicateur de sous-type pour le cancer de la tête et du cou, comprenant :
une première étape consistant à obtenir des données d'expression de miARN à partir d'un échantillon présentant un cancer de la tête et du cou ;
une seconde étape consistant à obtenir des données d'expression de miARN à partir d'une base de données relative au cancer de la tête et du cou ; et
une étape de sélection d'un miARN parmi les 15 % supérieurs de ceux dont les niveaux d'expression présentent une corrélation entre les niveaux d'expression des miARN de la première étape et de la seconde étape, dans lequel la base de données de la seconde étape contient des données d'expression capables d'identifier un sous-ensemble de cancer de la tête et du cou **caractérisé par** les données d'expression qui sont moyennées et ont un écart absolu moyen de 75 % ou moins,
dans lequel le miARN est de préférence choisi dans le groupe constitué par un premier groupe et un second groupe,
dans lequel le premier groupe est un ou plusieurs éléments quelconques sélectionnés dans le groupe constitué par miR-206, miR-127-5p, miR-432-5p, miR-409-5p, miR-145-3p, miR-497-5p, miR-195-5p, miR-150-5p, miR-140-3p, miR-214-3p, miR-125b-5p et miR-99a-5p, et
le second groupe est un ou plusieurs éléments quelconques sélectionnés dans le groupe constitué par miR-203a, miR-200a-5p, miR-200a-3p, miR-200b-5p, miR-200b-3p, miR-200c-3p, miR-141-3p, miR-205-5p, miR-183-5p, miR-106b-3p, miR-93-5p, miR-17-3p, miR-17-5p, miR-20a-5p, miR-92a-3p, miR-744-5p, miR-324-3p, miR-193a-5p, miR-149-5p, miR-1307-3p, miR-107, miR-103a-3p, miR-221-3p, miR222, miR-532-5p, miR-24-3p, miR-27b-5p, miR-27b-3p et miR-23b-3p,
dans lequel
(i) lorsque le miARN du premier groupe est régulé positivement, le sous-type de cancer de la tête et du cou est identifié comme étant un sous-type stromal,
(ii) lorsque le miARN du second groupe est régulé négativement, le sous-type de cancer de la tête et du cou est identifié comme étant un sous-type stromal,
(iii) lorsque le miARN du premier groupe est régulé négativement, le sous-type de cancer de la tête et du cou est identifié comme un sous-type tumoral, ou
(iv) lorsque le miARN du second groupe est régulé positivement, le sous-type de cancer de la tête et du cou est identifié comme un sous-type tumoral.
